(19) Europäisches Patentamt
European Patent Office
Office européen des brevets

(11) Veröffentlichungsnummer: **0 218 172**
**B1**

(12) # EUROPÄISCHE PATENTSCHRIFT

(45) Veröffentlichungstag der Patentschrift: **17.10.90**

(51) Int. Cl.[5]: **C07C 275/10**, C08G 18/38, C08G 18/50, C08G 65/28

(21) Anmeldenummer: **86113395.7**

(22) Anmeldetag: **29.09.86**

(54) **Oligoharnstoffpolyole, daraus erhaltene Polyetherpolyole und die Verwendung dieser Polyole im Isocyanat-Polyadditions-Verfahren.**

(30) Priorität: **08.10.85 DE 3535861**
**18.03.86 DE 3608962**

(43) Veröffentlichungstag der Anmeldung: **15.04.87 Patentblatt 87/16**

(45) Bekanntmachung des Hinweises auf die Patenterteilung: **17.10.90 Patentblatt 90/42**

(84) Benannte Vertragsstaaten: **BE DE FR GB IT SE**

(56) Entgegenhaltungen: **DE-A- 2 657 140**

(73) Patentinhaber: **BAYER AG, D-5090 Leverkusen 1 Bayerwerk(DE)**

(72) Erfinder: **Haas, Peter, Dr., Zwengenberger Strasse 43, D-5657 Haan 1(DE)**
Erfinder: **Sommerfeld, Claus-Dieter, Dr., Oberdreisbach Höhe 42, D-5203 Much(DE)**
Erfinder: **Weber, Hans-Ulrich, Dr., Neustrasse 19, D-4019 Monheim(DE)**

**EP 0 218 172 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Zur Herstellung von Polyolen, die insbesondere im Isocyanat-Polyadditions-Verfahren Verwendung finden, sind bislang eine Vielzahl von Startern beschrieben worden.

Als Starterspecies werden je nach angestrebter Funktionalität des Polyetherpolyols die unterschiedlichsten Verbindungen eingesetzt, um dann durch Addition von vornehmlich Oxiranderivaten die Eigenschaften und Molmasse der Polyetherpolyole einzustellen.

Starter für solche Polyole sind z.B. Di- oder Triole, wie Glykol, Glycerin, Trimethylolpropan, Pentaerythrit, Sucrose oder Glucose. Aber auch Harnstoff ist als Startmolekül verwendet worden, so z.B. in FR-PS 7 700 831, FR-PS 7 624 154, FR-PS 947 847, BE-PS 678 064, US-PS 4 180 131. Gegenüber den hochfunktionellen Startern hat Harnstoff den Nachteil einer niederen Funktionalität von üblicherweise 2.

In der DE-A 3 332 794 sind Mono-Tetra-hydroxyalkylmono-harnstoffe als einbaufähige Flammschutzmittel für Polyurethan-Integralschaumstoffe beschrieben.

Gegenstand der vorliegenden Erfindung sind neue Polyhydroxyalkyl-oligoharnstoffe der allgemeinen Formel (I)

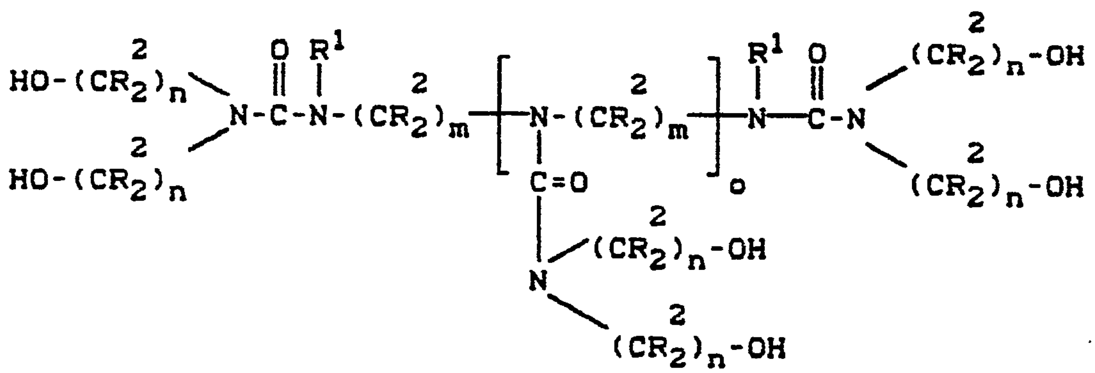

in welcher

die Reste $R^1$, gleich oder verschieden, für Wasserstoff oder einen $C_1$–$C_4$-Alkyl-Rest, bevorzugt Wasserstoff oder einen Methyl-Rest, und im Falle von o gleich Null die Reste $R^1$ auch für einen $C_1$–$C_6$-Alkylen-, bevorzugt $C_1$–$C_3$-Alkylen-, insbesondere $C_2$–$C_3$-Alkylen, insbesondere $C_2$-, Alkylen-Rest stehen können, wobei bei o gleich Null m immer eine ganze Zahl 2 oder 3 bedeutet,

die Reste $R^2$, gleich oder verschieden, für Wasserstoff oder einen $C_1$–$C_4$-Alkyl-Rest, vorzugsweise einen Methyl-Rest oder Wasserstoff stehen,

n eine ganze Zahl von 2 bis 6,

m eine Zahl von 2 bis 10,

o eine ganze Zahl von 0 bis 6, bevorzugt 0 bis 5, besonders bevorzugt 0 bis 3 und insbesondere 0 oder 1,

wobei Verbindungen der Formel

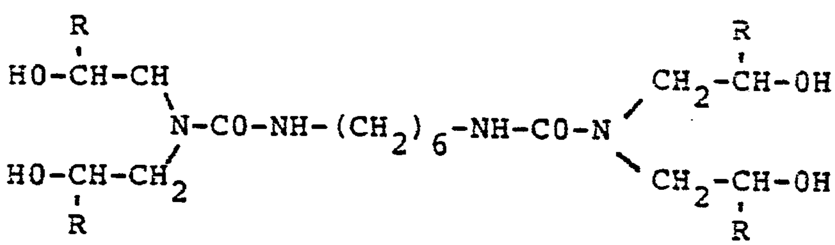

$R = H, CH_3$

ausgenommen sind, bedeuten,

oder der allgemeinen Formel (II)

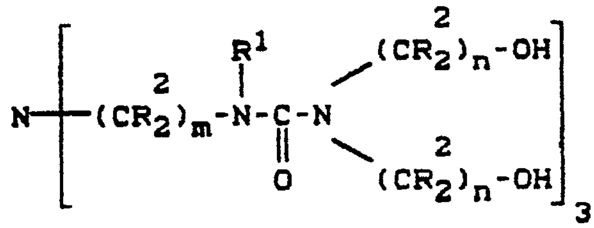

(II),

in welcher
$R^1$ Wasserstoff oder einen $C_1$–$C_4$-Alkyl-Rest, vorzugsweise Wasserstoff oder einen Methylrest,
die Reste $R^2$, gleich oder verschieden, Wasserstoff oder einen $C_1$–$C_4$-Alkyl-Rest, vorzugsweise Wasserstoff oder einen Methylrest,
n eine ganze Zahl von 2 bis 6 und
m eine ganze Zahl von 2 bis 10 bedeuten.
Bevorzugt sind Verbindungen, bei denen in den allgemeinen Formeln
n gleich 2 oder 3,
m gleich 2 oder 3,
o gleich 0 bis 3, insbesondere 0 oder 1,
$R^1$, $R^2$ Wasserstoff
bedeuten.

Die Erfindung betrifft auch Polyetherpolyole erhältlich durch Alkoxylierung der obengenannten Polyhydroxyalkyloligoharnstoffe.

Erfindungsgemäß bevorzugt sind dabei Polyetherpolyole erhältlich durch Alkoxylierung der erfindungsgemäßen Polyhydroxyalkyl-oligoharnstoffe mit Ethylenoxid und/oder Propylenoxid.

Diese Polyetherpolyole weisen bevorzugt eine OH-Zahl von 25 bis 700 auf.

Die Erfindung betrifft ferner auch die Verwendung der Polyhydroxyalkyl-oligoharnstoffe und der daraus erhältlichen Polyetherpolyole als Reaktionskomponente mit Isocyanaten zur Herstellung von Polyurethan(harnstoff)en beziehungsweise Polyisocyanuraten, vorzugsweise von Hartschaumstoffen auf Polyurethan(harnstoff)- oder Polyisocyanurat-Basis.

Die erfindungsgemäßen Polyhydroxyalkyl-oligoharnstoffe können z.B. durch Kondensation von Harnstoffderivaten der Formel (III)

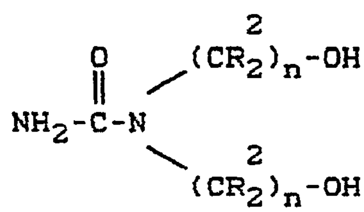

(III)

mit Aminen der allgemeinen Formel (IV) oder (V)

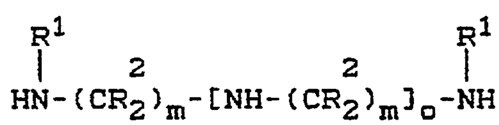

(IV)

und/oder

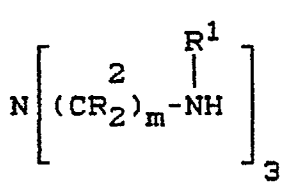

(V)

wobei n, m, $R^1$, $R^2$ und o die vorgenannte Bedeutung haben,
unter Austreiben von Ammoniak hergestellt werden.

Durch Alkoxylierung mit Oxiranderivaten wie Ethylenoxid, Propylenoxid, Butylenoxid oder Epichlorhydrin können diese hochfunktionellen Starter auf beliebige Molekulargröße bzw. Hydroxylzahlen eingestellt werden.

Ein Vorteil der erfindungsgemäßen Oligoharnstoffe ist weiterhin, daß diese Hydroxyalkyl-harnstoffe nicht nur an den Hydroxygruppen, sondern auch am Harnstoffwasserstoffatom alkoxyliert werden können, da diese Gruppe glatt acylierbar beziehungsweise alkoxylierbar ist.

Ausgangsstoffe zur Herstellung der erfindungsgemäßen Hydroxyalkyl-oligoharnstoffe sind aliphatische oder cyclophatische Diamine wie Imidazolidin, 1,3-Diazacycloheptan, Piperazin, Ethylendiamin, 1,2-Propylendiamin, 1,4-Diaminobutan, 1,5-Diaminopentan, 1,6-Diaminohexan, 1,8-Diaminoctan, 1,10-Diaminodecan, 1,4-Diaminocyclohexan, Isophorondiamin oder 4,4'- und/oder 2,4'- und/oder 2,2'-Diaminodicylohexylmethan sowie seine $C_1$-$C_4$-Mono- bistetraalkylderivate sowie Polyamine wie Diethylentriamin, Triethylentetramin, Tetraethylenpentamin, Pentaethylenhexamin oder Hexaethylenheptamin, sowie tertiäre Aminogruppen enthaltende Polyamine wie Tris-(3-amino-n-propyl)-amin, Tris-(2-aminoethyl)-amin, Tris-(4-amino-n-butyl)-amin oder Tris(2-amino-2-methyl-ethyl)-amin.

Die Verbindungen vom Typ der N,N-Bis-hydroxyalkylharnstoffe der allgemeinen Formel (III) werden z.B. durch Kondensation von Harnstoff mit Bis-hydroxyalkyl-aminen unter Austreiben von Ammoniak hergestellt.

Selbstverständlich kann die Kondensation auch in einem Einstufenverfahren erfolgen, so daß Harnstoff, Bis-hydroxyalkylamine sowie die Di- und Oligoamine direkt kondensiert werden.

Als Beispiele für die erfindungsgemäßen Polyhydroxyalkyloligoharnstoffe seien angeführt:

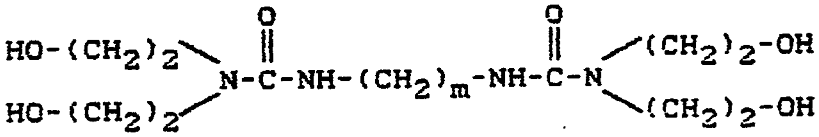

m= 2 bis 10 (mit Ausnahme von m = 6)

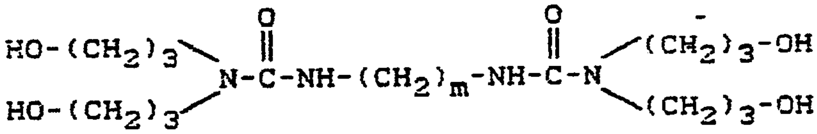

m= 2 bis 10

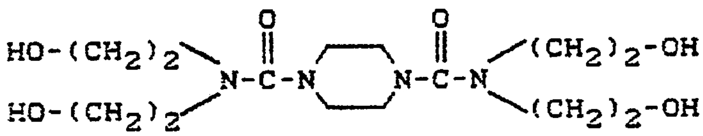

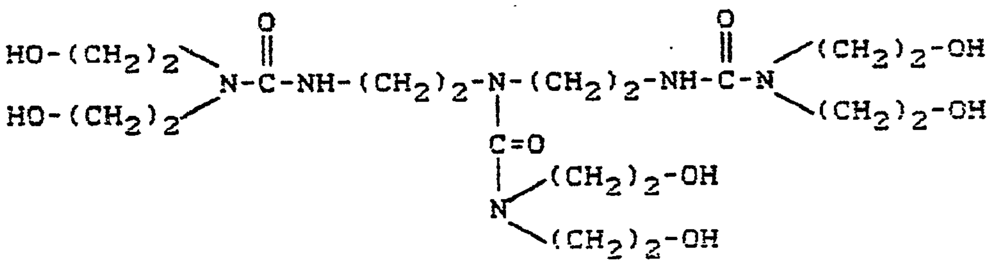

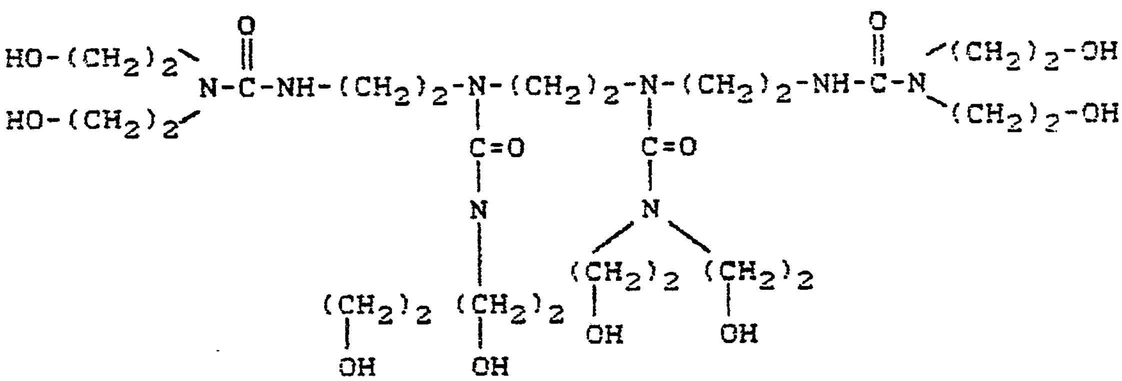

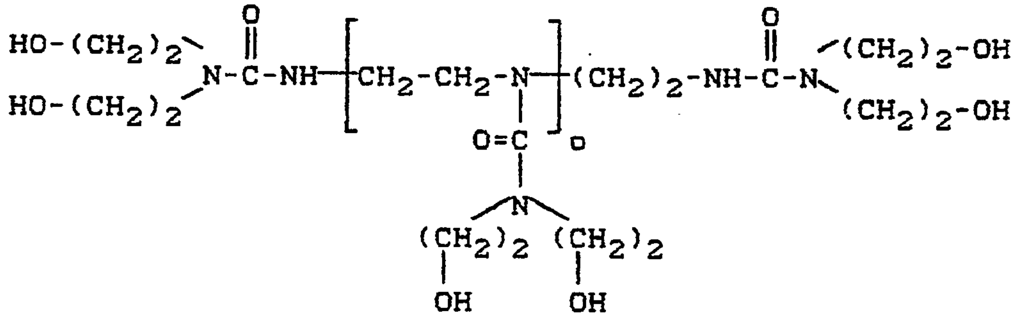

o = 3, 4 oder 5

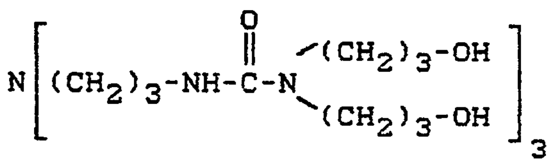

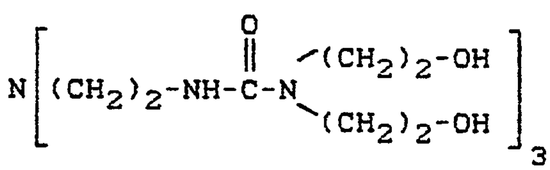

Die erfindungsgemäßen Polyhydroxyalkyl-oligoharnstoffe der allgemeinen Formel (I) und/oder (II) werden in der Regel unter üblichen Bedingungen mit Oxiranderivaten, bevorzugt Ethylenoxid und/oder Propylenoxid, in Mischung oder in sequentieller Form umgesetzt, wobei Polyetherpolyole mit Hydroxylzahlen zwischen 25 und 700, bevorzugt 250 und 600 erhalten werden; sie erfindungsgemäßen Oligoharnstoffe können jedoch auch ohne Alkoxylierung als Reaktionskomponente im Isocyanat-Polyadditionsverfahren eingesetzt werden.

Die erfindungsgemäßen Polyhydroxyalkyl-oligoharnstoffe und ihre Alkoxylierungsprodukte (Polyetherpolyole) verleihen nicht nur den Reaktivsystemen zur Schaumstoffbildung sondern auch Polyurethanschaumstoffen, vorzugsweise Polyurethanhartschaumstoffen, neue Eigenschaften, wie bei der Herstellung

- Autokatalyse, so daß auf Aktivatoren im Reaktivsystem verzichtet werden kann,
- eine hohe Schaumstabilität im sich bildenen Schaumstoff, so daß auf Stabilisatoren verzichtet werden kann,
- extreme Feinzelligkeit der Schaumstoffe auch ohne Zellregler oder Emulgatoren, bei den Schäumen
- Kaltverformbarkeit und
- thermoplastische Eigenschaften, d.h. sie ergeben z.B. thermoplastisch verformbare Hartschaumstoffe,

so daß sie wertvolle Polyolkomponenten im Sinne der Isocyanat-Polyadditonsreaktion darstellen. Selbstverständlich können auch Polyisocyanuratschaumstoffe mit den erfindungsgemäßen Polyolen hergestellt werden.

Zur Herstellung von Hartschaumstoffen, vorzugsweise Polyurethan(harnstoff)- und/oder Polyisocyanurat-Hartschaumstoffen, werden neben den erfindungsgemäßen Polyolen eingesetzt:

1. Aliphatische, cycloaliphatische, araliphatische, heterocyclische und besonders aromatische Di- und/oder Polyisocyanate, wie sie z.B. von W. Siefken in Justus Liebigs Annalen der Chemie, 562, Seiten 75-136 (1949), beschrieben werden, beispielsweise solche der Formel $Q(NCO)_n$, in der n = 2 bis 4, vorzugsweise 2 und Q einen aliphatischen Kohlenwasserstoffrest mit 2 bis 18, vorzugsweise 6 bis 12 C-Atomen, einen cycloaliphatischen Kohlenwasserstoffrest mit 4 bis 20, vorzugsweise 5 bis 11 C-Atomen, einen aromatischen Kohlenwasserstoffrest mit 6 bis 20, vozugsweise 6 bis 13 C-Atomen, oder einen araliphatischen Kohlenwasserstoffrest mit 8 bis 15, vorzugsweise 8 bis 13 C-Atomen bedeuten, z.B. solche Polyisocyanate wie sie in der DE-A 2 832 253, Seiten 10 bis 11, beschrieben werden. Besonders bevorzugt werden die technisch leicht zugänglichen Polyisocyanate, z.B. das 2,4- und/oder 2,6-Toluylendiisocyanat sowie beliebige Gemische dieser Isomeren ("TDI"), Diphenylmethan-diisocyanate (4,4'-und/oder 2,4'- und/oder 2,2'-Isomere), Polyphenyl-polymethylen-polyisocyanate, wie sie durch Anilin-Formaldehyd-Kondensation und anschließende Phosgenierung hergestellt werden ("rohes MDI") und "modifizierte Polyisocyanate", die z.B. Carbodiimidgruppen, Urethangruppen, Allophanatgruppen, Isocyanuratgruppen, Harnstoffgruppen und/oder Biuretgruppen aufweisen: insbesondere solche modifizierten Polyisocyanate, die sich vom 2,4- und/oder 2,6-Toluylendiisocyanat und bevorzugt vom 4,4'- und/oder 2,4'-Diphenylmethandiisocyanat ableiten.
Werden nur zweifunktionelle höhermolekulare Verbindungen und gegebenenfalls nur zweifunktionelle weitere, niedermolekulare Kettenverlängerungsmittel verwendet, so setzt man bevorzugt modifizierte Poly isocyanate mit einer Funktionalität von mehr als 2,0 ein bzw. verwendet tri- und/oder höherfunktionelle Polyisocyanate.

2. Ferner werden gegebenenfalls als Ausgangsmaterialien zur Polyurethanherstellung Verbindungen mit mindestens 2 gegenüber Isocyanaten reaktionsfähigen Wasserstoffatomen und einem Molekulargewicht von 18 bis 399 mitverwendet. Auch in diesem Fall versteht man hierunter Hydroxylgruppen und/oder Aminogruppen und/oder Thiolgruppen und/oder Carboxylgruppen und/oder Hydrazidgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gegenüber Isocyanaten reaktionsfähige Wasserstoffatome auf. Beispiele werden hierfür in der DE-A 2 832 253, Seiten 19-20, beschrieben. Genannt seien beispielsweise Wasser, Hydrazin, Ethylenglykol, Butandiol-1,4 Trimethylolpropan, Formit-Gemische oder Adipinsäuredihydrazid.

3. Als Copolyole der erfindungsgemäßen Polyharnstoffpolyole können gegebenenfalls Verbindungen mit mindestens zwei gegenüber Isocyanaten reaktionsfähigen Wasserstoffatomen von einem Molekulargewicht in der Regel von 400 bis 10.000 mitverwendet werden. Hierunter versteht man neben Aminogruppen, Thiogruppen oder Carboxylgruppen aufweisenden Verbindungen vorzugsweise Hydroxylgruppen aufweisende Verbindungen, insbesondere 2 bis 8 Hydroxylgruppen aufweisende Verbindungen, speziell solche vom Molekulargewicht 600 bis 6.000, vorzugsweise 1.500 bis 4.000, vorzugsweise aber 2 bis 4 Hydroxylgruppen aufweisende Polyester, Polyether, Polythioether, Polyacetale, Polycarbonate und Polyesteramide, wie sie für die Herstellung von homogenen und zellförmigen Polyurethanen an sich bekannt sind und wie sie z.B. in der DE-A 2 832 253,, Seiten 11 bis 18, beschrieben werden. Besonders bevorzugt sind Polyether, die durch Addition von einem oder mehreren Alkylenoxiden (Ethylenoxid und besonders Propylenoxid) an zwei- oder mehrwertige "Starter" (Propylenglykol, Glycerin, Sorbit, Formose, Triethanolamin, Trimethylolpropan) erhalten werden, sowie Polyether, welche Polyadditionsprodukte aus Diisocyanaten und Hydrazin und/oder Diaminen und/oder Glykolen oder Polymerisate und/oder Pfropfpolymerisate, vorzugsweise aus Styrol und Acrylnitril dispergiert oder gelöst enthalten. Die bevorzugten Polyether haben dabei eine mittlere Funktionalität über 2,0.

4. Gegebenenfalls werden Hilfs- und Zusatzmittel wie leichtflüchtige anorganische, jedoch bevorzugt organische Substanzen als Treibmittel, Katalysatoren der an sich bekannten Art, wie tertiäre Amine, Zinn(II)- und Zinn(IV)-Verbindungen, oberflächenaktive Zusatzstoffe, wie Emulgatoren und Schaumstabilisatoren, Reaktionsverzögerer, z.B. sauer reagierende Stoffe wie Salzsäure oder organische Säurehalogenide, ferner Zellregler der an sich bekannten Art wie Paraffine, Fettalkohole oder Dimethylpolysiloxane sowie Pigmente oder Farbstoffe der an sich bekannten Art, ferner Stabilisatoren gegen Alterungs-, Licht- und Witterungseinflüsse, Weichmacher und fungistatisch und bakteriostatisch wirkende Substanzen, sowie Füllstoffe zugesetzt. Diese gegebenenfalls mitzuverwendenden Hilfs- und Zusatzstoffe werden beispielsweise in der DE-A 2 732 292, Seiten 21 bis 24, ausführlich beschrieben. Weitere Beispiele der Hilfs- und Zusatzmittel werden im Kunststoffhandbuch, Band VII, herausgegeben von Vieweg und Hoechtlen, Carl-Hanser-Verlag, München 1966, auf den Seiten 103 bis 113, sowie im Kunststoffhandbuch Band VII, herausgegeben von Becker und Braun, Carl-Hanser-Verlag, München 1983 auf den Seiten 92 bis 111, beschrieben.
Mit den erfindungsgemäßen Harnstoffpolyolen hergestellte Hartschaumstoffe finden Verwendung als Dämmplatten, Sandwich-Elemente mit verschiedenen Deckschichten, als Ortschaumstoffe wie Spritzschaumstoffe oder nach dem Überschichtungsverfahren hergestellte Hartschaumstoffe, Sonnenkollektorfüllungen, Rohrisolierungen, als Füll-und Montageschaumstoffe und Blockschaumstoffe.
Ihre Herstellung erfolgt nach kontinuierlichen oder diskontinuierlichen Verfahren der Polyurethanverarbeitung wie z.B. Doppelbandtechnik, Spritz- oder Gießverfahren, mit Hoch- oder Niederdruck-Verschäumungsmaschinen.

Beispiele

Beispiel 1

$$a)\ NH_2-\overset{\overset{\displaystyle O}{\|}}{C}-N(CH_2-CH_2-OH)_2$$

Aus 1200 g (20 mol) Harnstoff und 2100 g (20 mol) Diethanolamin werden durch Austreiben von Ammoniak bei 100-120°C und Nachbehandlung im Wasserstrahlvakuum ein Reaktionsprodukt, das beim Abkühlen erstarrt, der oben angegebenen Formel hergestellt.

Ausbeute quantitativ, OH-Zahl 766 (ber. 750).

$$b)\ (HO-CH_2-CH_2)_2N-\overset{\overset{\displaystyle O}{\|}}{C}-NH-CH_2-CH_2-NH-\overset{\overset{\displaystyle O}{\|}}{C}-N(CH_2-CH_2-OH)_2$$

Aus 592 g (4 mol) der Verbindung gemäß a) und 120 g (2 mol) Ethylendiamin werden durch Erwärmen auf 130°C und Abspalten von Ammoniak sowie anschließende Behandlung im Wasserstrahlvakuum eine Verbindung der der oben angegebenen Formel hergestellt.

Ausbeute: 630 g, fast quantitativ
Viskosität: 5800 mPa.s (25°C)
OH-Zahl ber. für Tetrafunktionalität (nur OH-Gruppen): 695
Oh-Zahl*) ber. für Hexafunktionalität (OH-Gruppen plus NH-Gruppen aus der Harnstoffgruppe) 1040
OH-Zahl*) gef. 970

*) bei der OH-Zahl-Bestimmung werden auch NH-Gruppen aus der Harnstoffgruppe durch Acylierung miterfaßt.

Beispiel 2

$$(HO-CH_2-CH_2)_2N-\overset{\overset{\displaystyle O}{\|}}{C}-NH-CH_2-CH_2-\underset{\underset{\displaystyle N(CH_2-CH_2-OH)_2}{\underset{|}{\underset{\displaystyle C=O}{|}}}}{N}-CH_2-CH_2-NH-\overset{\overset{\displaystyle O}{\|}}{C}-N(CH_2-CH_2-OH)_2$$

Aus 1776 g (12 mol) der Verbindung nach Beispiel 1a) und 412 g (4 mol) Diethylentriamin werden durch Austreiben von Ammoniak bei 120-140°C und anschließender Behandlung im Vakuum eine Verbindung der oben angegebenen Formel hergestellt.

Ausbeute: 1975 g, fast quantitativ
OH-Zahl ber. für Hexafunktionalität 675
OH-Zahl*) ber. für Oktafunktionalität 902
OH-Zahl*) gef. 900
Viskosität 19.500 mPa.s (25°C)

Beispiel 3

180 g (3 Mol) Harnstoff, 315 g (3 m) Diethanolamin und 103 g (1 Mol) Diethylentriamin werden auf eine Temperatur von 110 bis 135°C erhitzt: nach ca. 10 Stunden wird auf 80°C abgekühlt und Wasserstrahlvakuum angelegt.

Ausbeute 494,5 g, quantitativ
Viskosität 28.000 m Pa.s (25°C)
OH-Zahl: 800

Beispiel 4

a) Aus 332,5 (2,5 mol) Diisopropanolamin und 150 g (2,5 mol) Harnstoff werden durch Austreiben von Ammoniak bei 120°C und anschließender Behandlung im Vakuum eine Verbindung der allgemeinen Formel

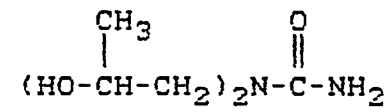

hergestellt.
Ausbeute quantitativ
OH-Zahl 640 (ber. 636)*).
*) siehe Beispiel 1 / Erläuterung

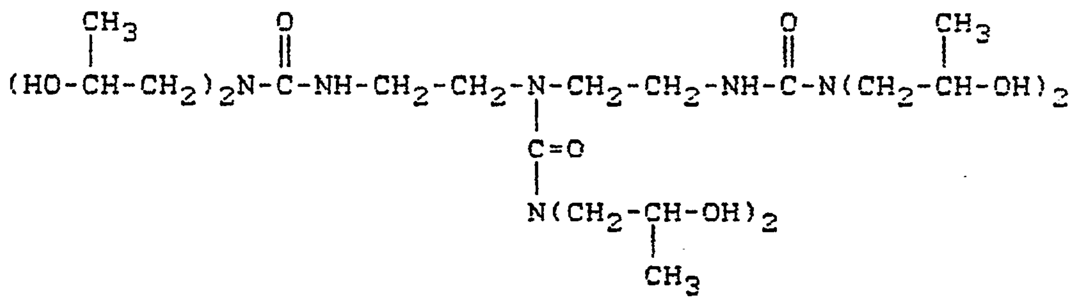

Aus 264 g (1,5 mol) der unter a) hergestellten Verbindung nach Beispiel 4 und 51,5 g (0,5 mol) Diethylentriamin werden durch Erhitzen auf 130°C und Austreiben von Ammoniak sowie anschließender Behandlung im Vakuum eine Verbindung der oben angegebenen Formel hergestellt.
Ausbeute quantitativ
OH-Zahl*) ber. für Octafunktionalität 770
OH-Zahl*) gef. 750
Viskosität 47.500 mPa.s (25°C)

Beispiel 5 (Polyetherpolyol)

2059 g der Verbindung nach Beispiel 2 werden mit 67 g einer 45 %igen KOH versetzt und anschließend 32 g Wasser abdestilliert, wonach bei 100°C innerhalb von 12 Std. 3941 g Propylenoxid aufgedrückt werden. Es wird mit 26,4 g Schwefelsäure in 600 g Wasser neutralisiert, eingeengt und abgesaugt.
Ausbeute 5420 g
OH-Zahl 386
Viskosität 1200 mPa.s (25°C)

Beispiel 6 (Polyetherpolyol)

20,6 kg der Verbindung nach Beispiel 2) werden mit 670 g einer 45 %igen KOH versetzt und anschließend Wasser abdestilliert, wonach bei 110°C innerhalb von 13 Std. 21,65 kg Propylenoxid aufgedrückt werden. Es wird mit 264 g Schwefelsäure in 6000 g Wasser neutralisiert, eingeengt und abgesaugt.
Ausbeute 40,3 kg
OH-Zahl 439
Viskosität 2175 mPa.s (25°C)
Wassergehalt 0,05 %

Beispiel 7 (Polyetherpolyol)

2060 g der Verbindung nach Beispiel 1b) werden mit 67 g einer 45%igen KOH versetzt und eingeengt, dann bei 110°C innerhalb von 10 Std. mit 1810 g Propylenoxid versetzt. Es wird mit 26,4 g Schwefelsäure in 700 g Wasser neutralisiert, eingeengt und abgesaugt.
Ausbeute 3,8 kg, OH-Zahl 395;

Beispiel 8 (Polyurethanhartschaumstoff)

98 g des erfindungsgemäßen Polyetherpolyols nach Beispiel 6, 1,5 g Wasser, 2,0 g eines Schaumstabilisators nach der DE-OS 2 029 293 (Silicon-Schaumstabilisator ®Tegostab B 8421 der Goldschmidt AG,

Essen) und 39 g Trifluormonochlormethan als Treibmittel werden gemischt.

Dieses Gemisch wird mit 133 g rohem 4,4'-Diisocyanatodiphenylmethan (NCO-Gehalt 31%) verschäumt. Durch die autokatalytische Wirkung des erfindungsgemäßen Polyetherpolyols entsteht ein amin-geruchsfreier, feinzelliger Hartschaumstoff mit sehr glatter Oberfläche. Durch das schlechte Luftlösevermögen weist der Schaumstoff keine "Wurmlöcher" auf.

Der erhaltene Schaumstoff hat folgende physikalische Daten:
Abbindezeit 75 sec.
Rohdichte 20 kg/m³ (Freischaum)
Rohdichte 31,5 kg/m³ (Formschaum)
Druckfestigkeit 0,11 MPa (für RD = 31,5 kg/m³)

<u>Beispiel 9</u> (Polyurethanhartschaumstoff)

98,5 g des erfindungsgemäßen Polyetherpolyols nach Beispiel 6, 1,5 g Wasser und 39 g Trifluormonochlormethan werden gemischt. Dieses Gemisch wird mit 133 g rohem 4,4'-Diisocyanatodiphenylmethan (NCO-Gehalt 31%) verschäumt.

Es entsteht ein zäher Hartschaumstoff mit einem feinen, überraschend gleichmäßigem Zellbild, hergestellt ohne Schaumstabilisator und Aktivator.
Physikalische Daten:
Abbindezeit 70 sec.
Rohdichte 21 kg/m³

<u>Beispiel 10</u> (Polyurethanhartschaumstoff)

68,5 g des erfindungsgemäßen Polyetherpolyols nach Beispiel 6, 29,6 g eines Polyetherpolyols, das durch Anlagerung von Propylenoxid an eine Mischung aus Sucrose, Propylenglykol und Wasser im Gewichtsverhältnis 75:15:10 und einer OH-Zahl von 470 erhalten worden ist, 1,9 g Wasser, 0,2 g eines Schaumstabilisators nach der DE-OS 2 029 293 (Silicon-Schaumstabilisator ®Tegostab B 8421 der Goldschmidt AG, Essen), 09 g Dimethylcyclohexylamin und 36 g Trifluormonochlormethan werden gemischt.

Dieses Gemisch wird mit 144 g rohem 4,4'-Diisocyanatodiphenylmethan (NCO-Gehalt 31%) verschäumt. Es entsteht ein gut fließendes Rohstoffgemisch sowie ein feinzelliger harter Schaumstoff mit sehr glatter Oberfläche.
Physikalische Daten:
Abbindezeit 75 sec.
Rohdichte 21 kg/m³ (Freischaum)
Rohdichte 32 kg/m³ (Formschaum)
Druckfestigkeit 0,11 MPa (für RD = 32 kg/m³)

<u>Beispiel 11</u> (Polyurethanhartschaumstoff)

96,5 g des erfindungsgemäßen Polyetherpolyols nach Beispiel 6, 1,5 g Wasser, 2 g eines Schaumstabilisators nach der DE-OS 2 029 293 (Silicon-Schaumstabilisator ®Tegostab B 8421 der Goldschmidt AG, Essen), 0,5 g N,N,N',N'',N''-Pentamethyldiethylentriamin, 5 g eines 98:2 Gemisches aus Dimethylcyclohexylamin und Dibutylzinndilaurat und 39 g Trifluormonochlormethan werden gemischt.

Dieses Gemisch wird mit 133 g rohem 4,4'-Diisocyanatodiphenylmethan (NCO-Gehalt 31%) vermischt und anschließend versprüht. Der gespritzte Hartschaumstoff ist sehr feinzellig und zeigt eine gute Lagenhaftung sowie eine glatte Oberfläche.
Physikalische Daten:
Abbindezeit 10 sec.
Rohdichte 35 kg/m³
Druckfestigkeit 0,19 MPa
Offenzelligkeit (nach ASTM-D-1940) 7 Vol-%
Querzugversuch (Festigkeit) 0,4 MPa

<u>Beispiel 12</u> (Polyisocyanurathartschaumstoff)

98 g des erfindungsgemäßen Polyetherpolyols nach Beispiel 5, 1 g eines Schaumstabilisators nach der DE-OS 2 029 293 (Silicon-Schaumstabilisator ®Tegostab B 8421 der Goldschmidt AG, Essen), 21 g einer 25%igen Lösung von Kaliumacetat in Diethylenglykol und 40 g Trifluormonochlormethan werden gemischt.

Dieses Gemisch wird mit 198 g rohem 4,4'-Diisocyanatodiphenylmethan (NCO-Gehalt 31%) verschäumt. Es entsteht feinzelliger, harter Polyisocyanuratschaumstoff.
Physikalische Daten:
Abbindezeit 80 sec.
Rohdichte 35 kg/m³ (Freischaum)

Beispiel 13 (Polyisocyanuratschaumstoff)

98 g des erfindungsgemäßen Polyetherpolyols nach Beispiel 6, 1 g eines Schaumstabilisators nach der DE-OS 2 029 293 (Silicon-Schaumstabilisator ®Tegostab B 8421 der Goldschmidt AG, Essen), 1 g einer Lösung von Kaliumacetat in Diethylenglykol, 15 g von Tris-β-chlorethylphosphat und 40 g Trifluormonochlormethan werden gemischt.

Dieses Gemisch wird mit 206 g rohem 4,4'-Diisocyanatodiphenylmethan (NCO-Gehalt 31%) verschäumt. Es entsteht ein feinzelliger Polyisocyanuratschaumstoff.

Physikalische Daten:
Abbindezeit 90 sec.
Rohdichte 40 kg/m³ (Freischaum)
Rohdichte 43 kg/m³ (Formschaum)
Druckfestigkeit 0,26 MPa (für RD = 43 kg/m³)
Offenzelligkeit (nach ASTM-D-1940) 8 Vol.-%

Beispiel 14 (Polyurethanhartschaumstoff)

30 g des erfindungsgemäßen Polyetherpolyols nach Beispiel 6, 35 g eines Polyesterpolyols der Hydroxylzahl 200, das durch Reaktion von einem 80:20 Gemisch aus Phthalsäureanhydrid und Adipinsäure mit Diethylenglykol erhalten worden ist, 30 g eines Polyetherpolyols der Hydroxylzahl 180, das durch Anlagerung von Ethylenoxid an Propylenglykol erhalten worden ist, 2,8 g Wasser und 0,3 eines Schaumstabilisators nach der DE-OS 2 029 293 (Silicon-Schaumstabilisator ®Tegostab B 8421 der Goldschmidt AG, Essen) werden gemischt.

Dieses Gemisch wird mit 150 g rohem 4,4'-Diisocyantodiphenylmethan (NCO-Gehalt 31%) verschäumt. Es entsteht ein zäher, feinzelliger Hartschaumstoff, der thermoplastisch verformbar ist.

Physikalische Daten:
Abbindezeit 80 sec.
Rohdichte 45 kg/m³ (Freischaum)
Heizzeit (bei 170°C) 3 min zur thermoplastischen Verformung

Beispiel 15 (Polyurethanhartschaumstoff)

57,6 g eines Polyetherpolyols der Hydroxylzahl 380, das durch Anlagerung von Propylenoxid an eine Mischung aus Sucrose, Propylenglykol und Wasser erhalten worden ist, 28,8 g des erfindungsgemäßen Polyetherpolyols nach Beispiel 6, 9,6 g Polyetherpolyol mit einer OH-Zahl von 650, das durch Anlagerung von Propylenoxid an Ethylendiamin erhalten worden ist, 2 g Wasser, 2 g eines Schaumstabilisators nach der DE-OS 2 029 293 (Silicon-Schaumstabilisator ®Tegostab B 8421 der Goldschmidt AG, Essen), 1,7 g Dimethylcyclohexylamin und 35 g Trifluormonochlormethan werden gemischt.

Dieses Gemisch wird mit 136 g rohem 4,4'-Diisocyanatodiphenylmethan (NCO-Gehalt 31%) verschäumt. Es entsteht ein sehr feinzelliger, harter Schaumstoff mit sehr glatter Oberfläche.

Physikalische Daten:
Abbindezeit 80 sec.
Rohdichte 21 kg/m³ (Freischaum)

**Patentansprüche**

1. Polyhydroxyalkyl-oligoharnstoffe der allgemeinen Formel I

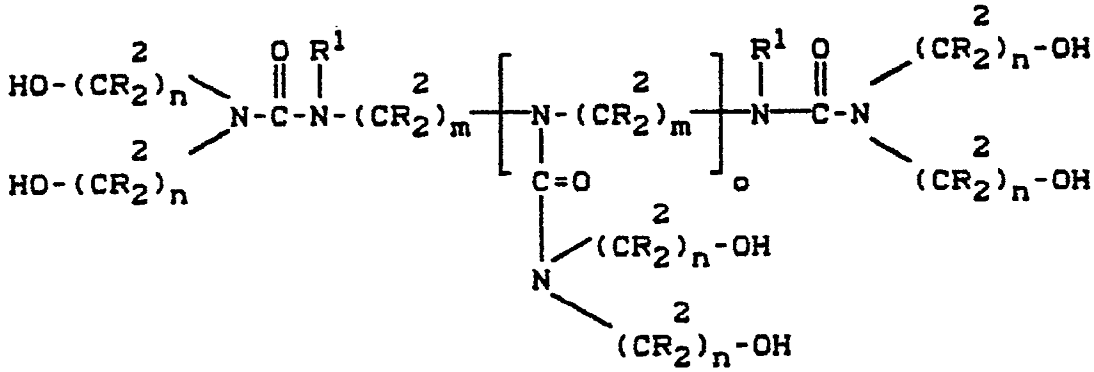

in welcher
die Reste $R^1$, gleich oder verschieden, für Wasserstoff oder einen $C_1$–$C_4$-Alkyl-Rest, bevorzugt Wasserstoff oder einen Methyl-Rest, und im Falle von o gleich Null die Reste $R^1$ auch für einen $C_1$–$C_6$-Alkylen-, bevorzugt $C_1$–$C_3$-Alkylen, insbesondere $C_2$–Alkylen-Rest stehen können, wobei bei o gleich Null m immer eine ganze Zahl 2 oder 3 bedeutet,
die Reste $R^2$, gleich oder verschieden, für Wasserstoff oder einen $C_1$–$C_4$-Alkyl-Rest, vorzugsweise einen Methyl-Rest oder Wasserstoff stehen,
n eine ganze Zahl von 2 bis 6
m eine ganze Zahl von 2 bis 10,
o eine ganze Zahl von 0 bis 6, bevorzugt 0 bis 5, besonders bevorzugt 0 bis 3 und insbesondere 0 oder 1,
wobei Verbindungen der Formel

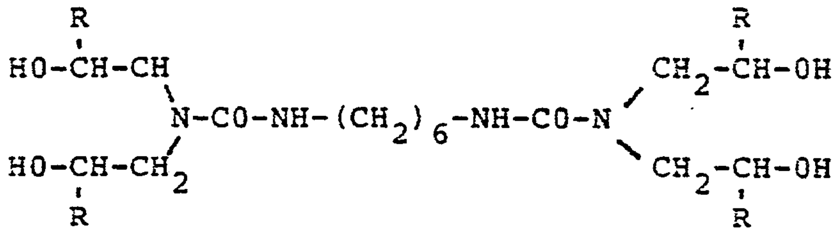

$R = H, CH_3$

ausgenommen sind, bedeuten,
oder der allgemeinen Formel (II)

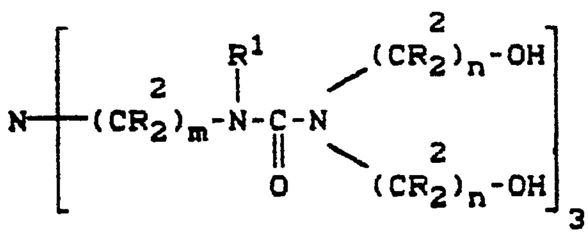

(II),

in welcher
$R^1$ Wasserstoff oder einen $C_1$–$C_4$-Alkyl-Rest, vorzugsweise Wasserstoff oder einen Methylrest,
die Reste $R^2$, gleich oder verschieden, Wasserstoff oder einen $C_1$–$C_4$-Alkyl-Rest, vorzugsweise Wasserstoff oder einen Methylrest,
n eine ganze Zahl von 2 bis 6 und
m eine ganze Zahl von 2 bis 10 bedeuten.

2. Polyhydroxyalkyl-oligoharnstoffe gemäß Anspruch 1, dadurch gekennzeichnet, daß in den allgemeinen Formeln
n gleich 2 oder 3,
m gleich 2 und 3,
o eine ganze Zahl von 0 bis 6, bevorzugt 0 bis 3, insbesondere 0 oder 1, und
die Reste $R^1$ und $R^2$ Wasserstoff bedeuten.

3. Polyhydroxyalkyl-oligoharnstoff der Formel

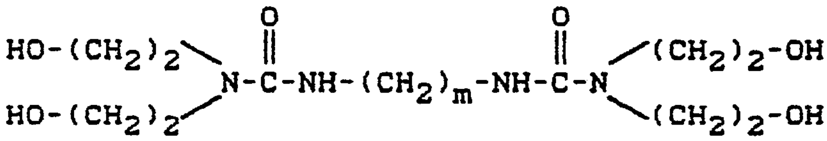

m= 2 bis 10 (mit Ausnahme von m = 6)

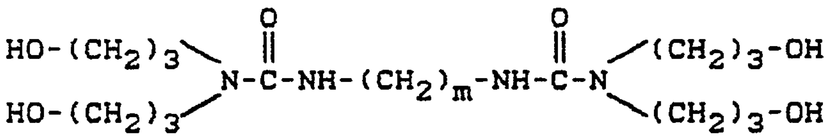

m= 2 bis 10

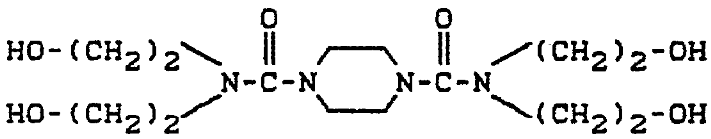

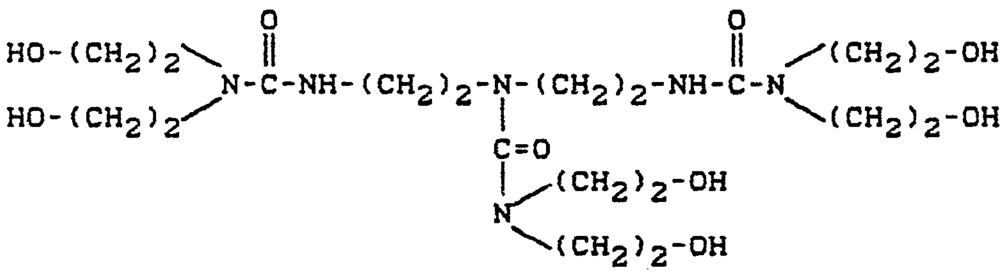

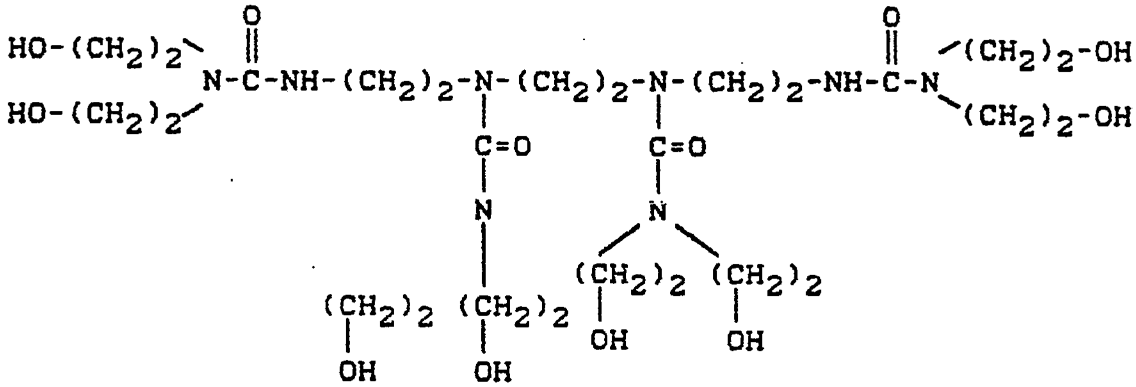

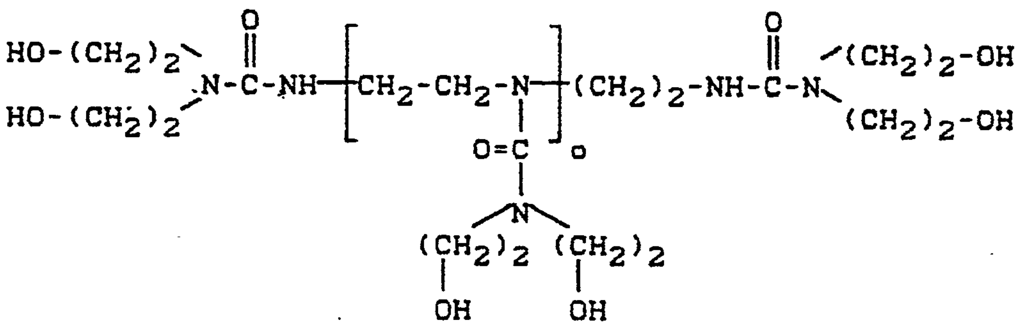

o = 3, 4 oder 5

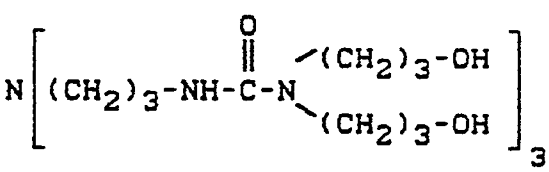

oder

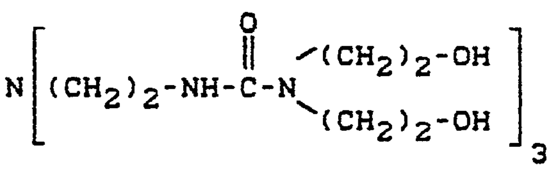

4. Polyetherpolyole erhältlich durch Alkoxylierung von Polyhydroxyalkyl-oligoharnstoffen nach Anspruch 3.

5. Polyetherpolyole nach Anspruch 4, erhältlich durch Alkoxylierung mit Ethylenoxid und/oder Propylenoxid.

6. Polyetherpolyole nach Anspruch 4 oder 5 mit einer OH-Zahl von 25 bis 700.

7. Verwendung der Verbindungen nach Ansprüchen 1 bis 6 als Reaktionskomponente im Isocyanat-Polyadditionsverfahren.

8. Verwendung nach Anspruch 7 in der Hartschaumstoffherstellung.

## Claims

1. Polyhydroxyalkyl oligoureas corresponding to general formula I

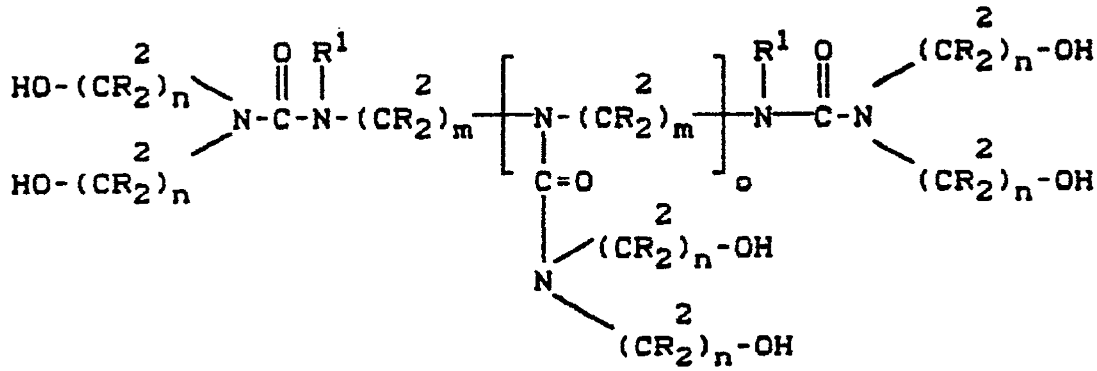

in which
the substituents $R^1$ may be the same or different and represent hydrogen or a $C_{1-4}$ alkyl radical, preferably hydrogen or a methyl radical, and where o = 0, the substituents $R^1$ may even be a $C_{1-6}$ alkylene, preferably a $C_{1-3}$ alkylene, more preferably a $C_{2-3}$ alkylene and, most preferably, a $C_2$ alkylene radical; where o = 0, m must always be an integer of 2 or 3,
the substituents $R^2$ may be the same or different and represent hydrogen or a $C_{1-4}$ alkyl radical, preferably a methyl radical or hydrogen,
n is an integer of 2 to 6,
m is an integer of 2 to 10,
o is an integer of 0 to 6, preferably 0 to 5, more preferably 0 to 3 and most preferably 0 or 1,
not including compounds having the formula

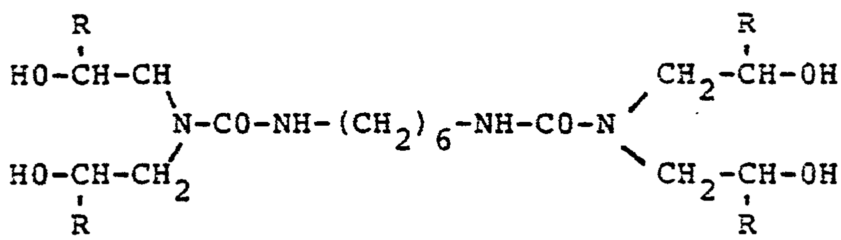

$R = H, CH_3$

or to general formula (II)

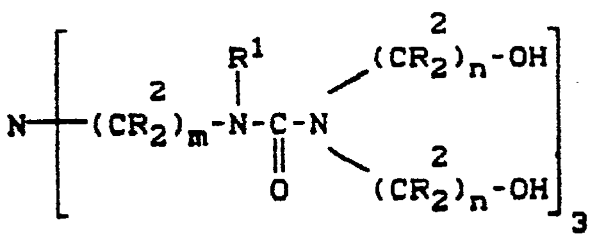 (II),

in which
$R^1$ is hydrogen or a $C_{1-4}$ alkyl radical, preferably hydrogen or a methyl radical,
the substituents $R^2$ may be the same or different and represent hydrogen or a $C_{1-4}$ alkyl radical, preferably hydrogen or a methyl radical,
n is an integer of 2 to 6 and
m is an integer of 2 to 10.

2. Polyhydroxyalkyl oligoureas as claimed in claim 1, characterized in that, in the general formulae,
n is 2 or 3,
m is 2 and 3,

o is an integer of 0 to 6, preferably 0 to 3 and more preferably 0 or 1 and the substituents $R^1$ and $R^2$ represent hydrogen.

3. Polyhydroxyalkyl oligourea corresponding to the formula

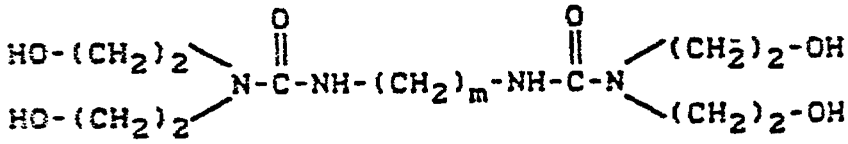

m= 2 to 10 (except for m = 6)

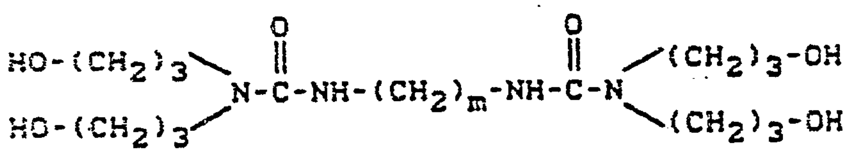

m= 2 to 10

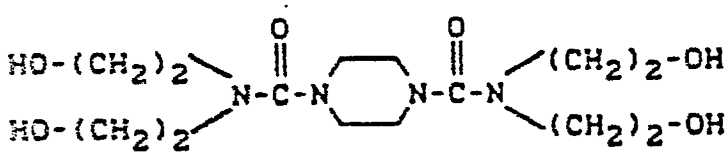

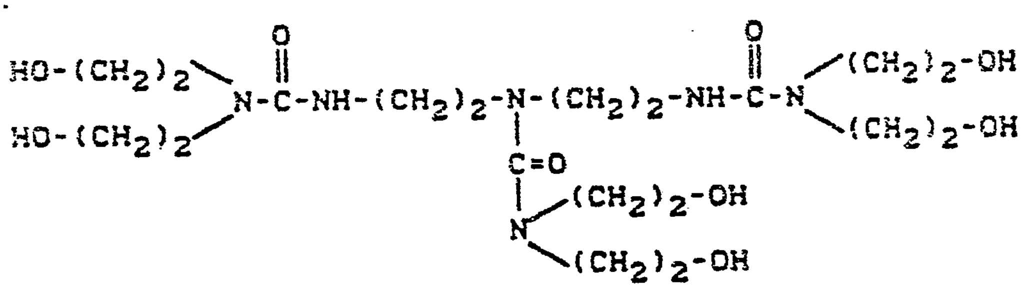

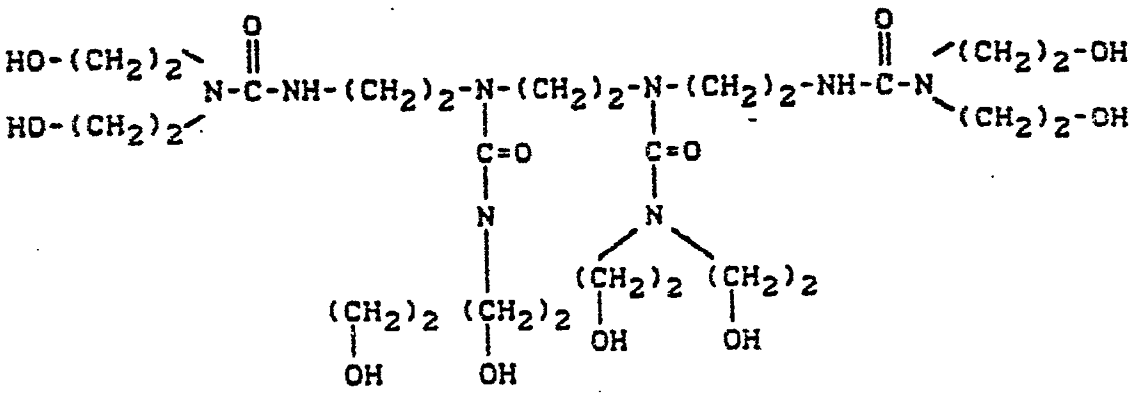

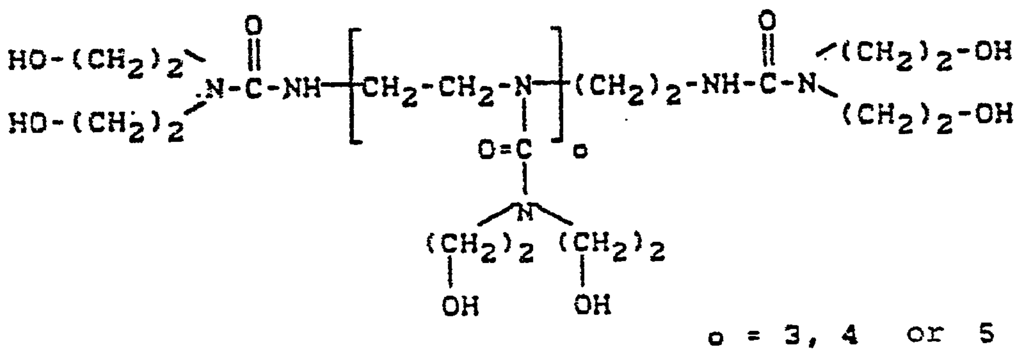

o = 3, 4 or 5

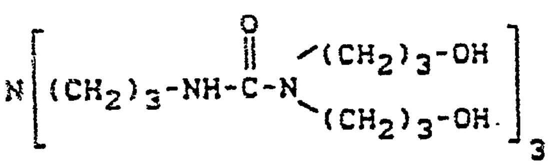

or

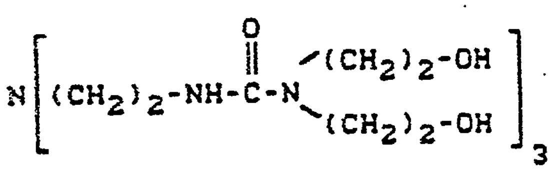

4. Polyether polyols obtainable by alkoxylation of the polyhydroxyalkyl oligoureas claimed in claims 1 to 3.

5. Polyether polyols as claimed in claim 4 obtainable by alkoxylation with ethylene oxide and/or propylene oxide.

6. Polyether polyols as claimed in claim 4 or 5 having an OH value of 25 to 700.

7. The use of the compounds claimed in claims 1 to 6 as a reaction component in the isocyanate polyaddition process.

8. The use claimed in claim 7 in the production of rigid foams.

**Revendications**

1. Polyhydroxyalkyl-oligo-urées de formule générale I

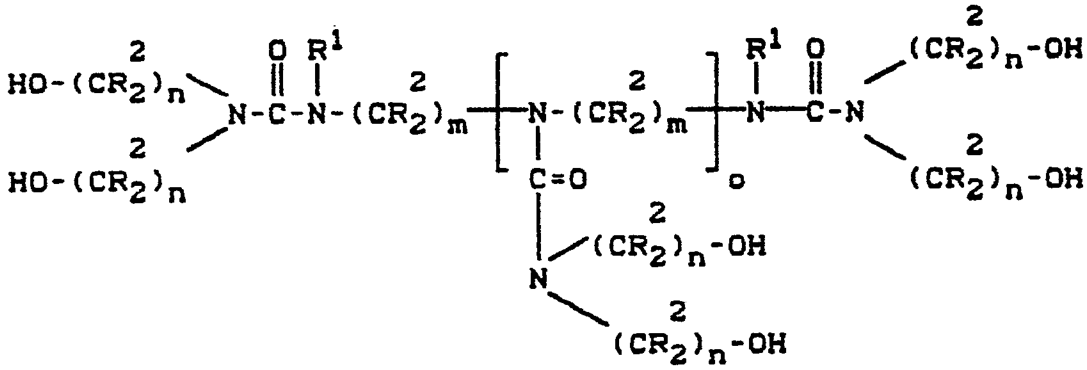

dans laquelle
les restes $R^1$, identiques ou différents, représentent l'hydrogène ou un reste alkyle en $C_1$ à $C_4$, de préférence l'hydrogène ou un reste méthyle, et au cas où o est égal à 0, les restes $R^1$ peuvent aussi représenter un reste alkylène en $C_1$ à $C_6$, de préférence alkylène en $C_1$ à $C_3$, notamment alkylène en $C_2$ ou $C_3$, plus spécialement alkylène en $C_2$, et lorsque o est égal à 0, m désigne toujours le nombre entier 2 ou 3,
les restes $R^2$, identiques ou différents, représentent l'hydrogène ou un reste alkyle en $C_1$ à $C_4$, de préférence un reste méthyle ou l'hydrogène,
n est un nombre entier de 2 à 6,
m est un nombre entier de 2 à 10,
o est un nombre entier de 0 à 6, de préférence de 0 à 5, notamment de 0 à 3 et plus particulièrement 0 ou 1,
à l'exception des composés de formule

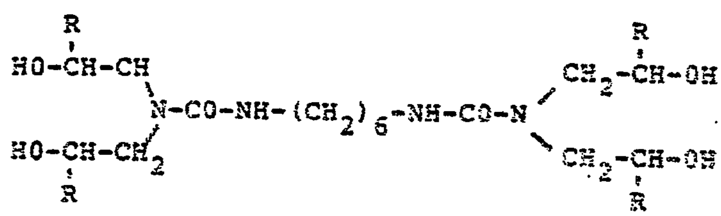

ou de formule générale (II)

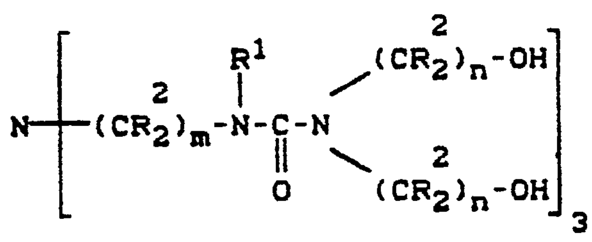

(II),

dans laquelle
$R^1$ est l'hydrogène ou un reste alkyle en $C_1$ à $C_4$, de préférence l'hydrogène ou un reste méthyle,
les restes $R^2$, identiques ou différents, représentent l'hydrogène ou un reste alkyle en $C_1$ à $C_4$, de préférence l'hydrogène ou un reste méthyle,
n est un nombre entier de 2 à 6 et
m est un nombre entier de 2 à 10.

2. Polyhydroxyalkyl-oligo-urées suivant la revendication 1, caractérisées en ce que dans les formules générales
n a la valeur 2 ou 3
m a les valeurs 2 et 3
o est un nombre entier de 0 à 6, de préférence de 0 à 3, notamment 0 ou 1, et

les restes $R^1$ et $R^2$ sont de l'hydrogène.

3. Polyhydroxyalkyl-oligo-urée de formule

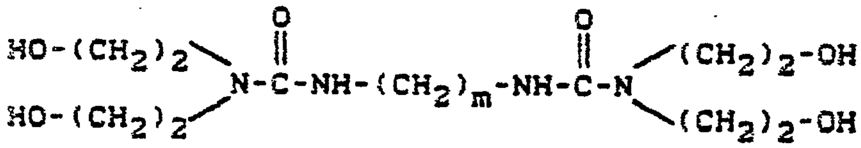

m= 2 à 10 (à l'exception de m=6)

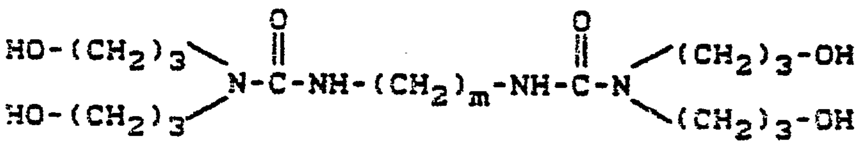

m= 2 à 10

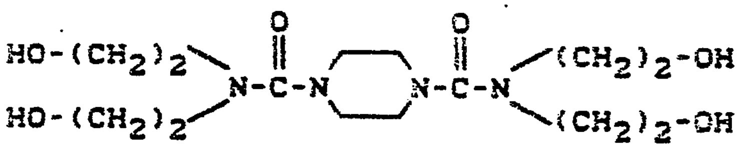

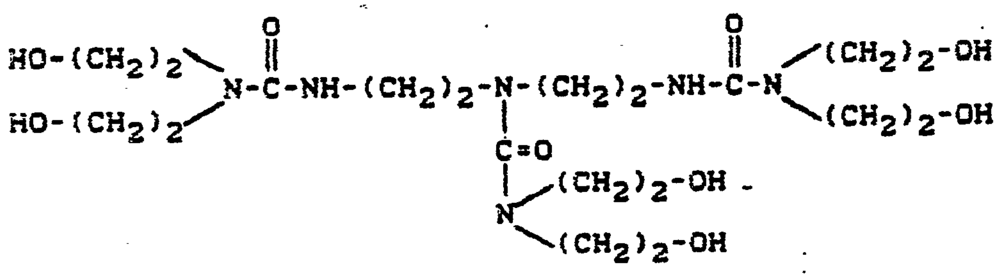

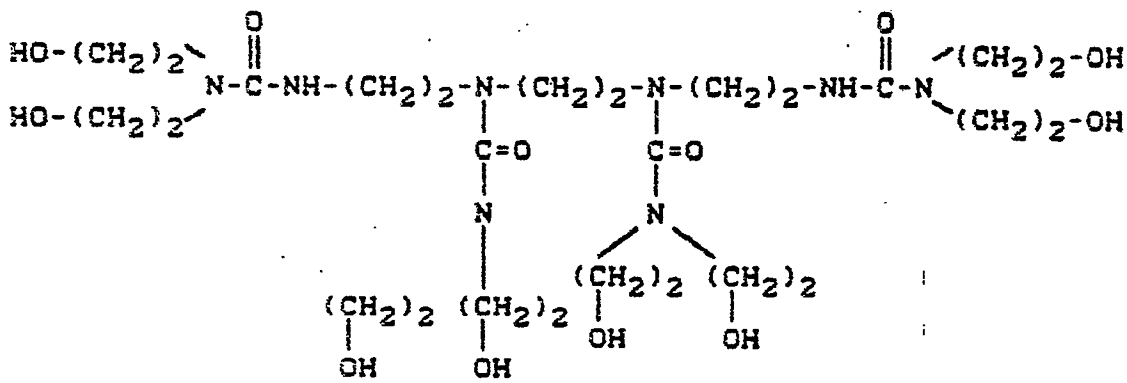

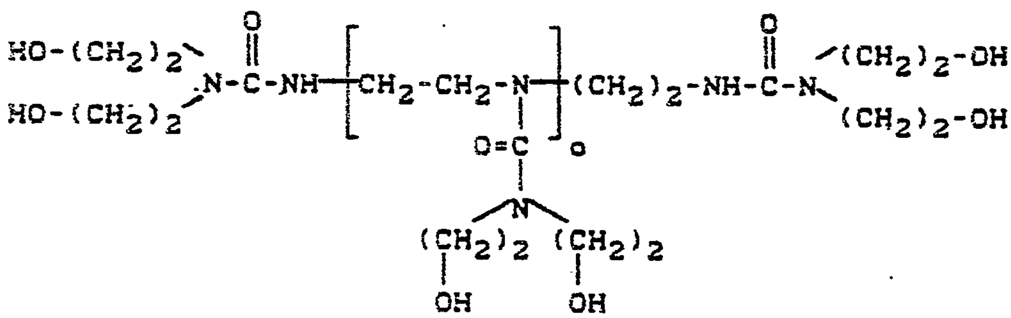

o = 3, 4 ou 5

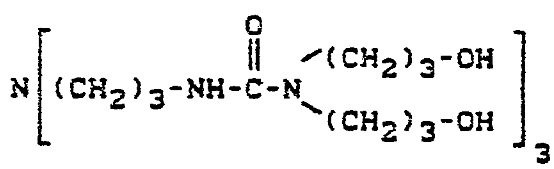

ou

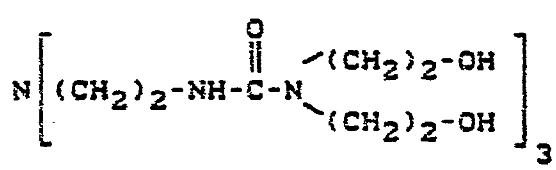

4. Polyétherpolyols obtenus par alkoxylation de polyhydroxyalkyl-oligo-urées suivant les revendications 1 à 3.

5. Polyétherpolyols suivant la revendication 4, obtenus par alkoxylation avec l'oxyde d'éthylène et/ou l'oxyde de propylène.

6. Polyétherpolyols suivant la revendication 4 ou 5, ayant un indice d'hydroxyle de 25 à 700.

7. Utilisation des composés suivant les revendications 1 à 6 comme composant réactionnel dans le procédé de polyaddition d'isocyanate.

8. Utilisation suivant la revendication 7 dans la production d'une mousse dure.